# FASCICULE DE BREVET EUROPEEN

(11) **EP 0 879 222 B1**
(45) Date de publication et mention de la délivrance du brevet: **14.03.2001**
(21) Numéro de dépôt: 96943195.6
(22) Date de dépôt: 24.12.1996
(51) Int. Cl.: C07C 53/21, C07C 51/34, C07C 303/16

(54) **PROCEDE DE PREPARATION D'ACIDES PERFLUOROALCANECARBOXYLIQUES ET PERFLUOROALCANESULFONIQUES**
VERFAHREN ZUR HERSTELLUNG VON PERFLUORALKANCARBON- UND PERFLUORALKANSULFONSÄURE
METHOD FOR PREPARATION OF PERFLUOROALCANECARBOXYLIC AND PERFLUOROALCANESULFONIC ACIDS

(30) Priorité: 27.12.1995 FR 9515558
(43) Date de publication de la demande: 25.11.1998
(73) Titulaire: ELECTRICITE DE FRANCE, 75008 Paris Cedex 08 (FR); CENTRE NATIONAL DE LA RECHERCHE SCIENTIFIQUE, 75016 Paris Cédex (FR)
(72) Inventeur: MARZOUK, Hatem, F-77210 Avon (FR); PETIT, Laurent, F-77300 Fontainebleau (FR); TORDEUX, Marc, F-92330 Sceaux (FR); BERECIBAR, Amaya, F-92160 Antony (FR); WAKSELMAN, Claude, F-75016 Paris (FR)
(74) Mandataire: Boulinguiez, Didier
(86) Numéro de dépôt international: FR9602079
(87) Numéro de publication internationale: WO9724309

(56) Documents cités:
- EP-A- 0 286 914
- DE-A- 2 556 844
- GB-A- 758 419
- US-A- 3 833 626
- US-A- 5 420 316
- CHEMICAL ABSTRACTS, vol. 84, no. 25, 21 Juin 1976 Columbus, Ohio, US; abstract no. 179636, UKIHASHI,HIROSHI ET AL.: "POLYFLUOROCARBOXYLIC ACIDS" page 522; colonne 2; XP002011593 & JP 75 157 314 A (ASAHI GLASS CO.,LTD.) 19 Décembre 1975

## Description

L'invention concerne un nouveau procédé de préparation d'acides perfluoroalcanecarboxyliques et perfluoroalcanesulfoniques.

Les acides perfluoroalcanecarboxyliques et perfluoroalcanesulfoniques jouent un rôle important dans l'industrie chimique d'aujourd'hui ("Organofluorine Chemistry, Principles and Commercial Applications", R.E. Banks, B.E. Smart, J.C. Tatlow, Eds., Plenum Press, New York 1994).

Les acides perfluoroalcanecarboxyliques et perfluoroalcanesulfoniques trouvent une grande diversité d'utilisations dans l'industrie chimique, aussi bien en tant que produits intermédiaires de synthèse qu'en tant que produits finis.

Les acides perfluoroalcanecarboxyliques de chaîne longue et leurs sels sont de bons agents tensioactifs, pouvant réduire de façon importante la tension à la surface de l'eau, des solutions aqueuses et des liquides organiques, même en concentrations basses. Ces acides et leurs dérivés peuvent également être utilisés comme agents émulsifiants, dispersants et moussants.

Les acides perfluoroalcanesulfoniques de chaîne courte servent de produits intermédiaires dans la préparation des sulfonamides. Ces derniers sont employés comme régulateurs de croissance des plantes et comme herbicides.

Les acides perfluoroalcanesulfoniques sont également utilisés en tant qu'agents antistatiques et antisalissants pour des substrats poreux tels que le papier et les textiles.

Des méthodes pour la préparation des acides perfluoroalcanecarboxyliques et perfluoroalcanesulfoniques sont déjà connues. Ainsi, ces acides sont généralement préparés soit par fluoration électrochimique, soit par l'utilisation d'agents chimiques à fort pouvoir oxydant. Cependant, ces méthodes ont chacune leurs inconvénients.

La technique de la fluoration électrochimique est délicate et onéreuse en raison des dangers liés à l'emploi du fluorure d'hydrogène liquide et de la corrosion des cellules d'électrolyse occasionnée par le milieu.

Les voies d'oxydation par agents chimiques comprennent l'action de l'oléum à chaud sur les iodures de perfluoroalkyle. L'utilisation de l'oléum (acide sulfurique très concentré contenant du trioxyde de soufre SO₃) est également dangereuse en raison de ses propriétés corrosives. De plus, cette méthode entraîne la dégradation partielle de la chaîne carbonée, conduisant à l'obtention d'un mélange d'acides fluorés homologues.

Le brevet JP.75157314 décrit un procédé de préparation d'acides polyfluoroalkyliques par ozonolyze d'un composé éthylénique insaturé contenant des radicaux polyfluoroalkyles déterminés.

Le procédé selon la présente invention utilise de l'ozone pour la préparation des acides perfluoroalcanecarboxyliques et perfluoroalcanesulfoniques. L'ozone permet une oxydation douce et sélective des produits de départ, remédiant ainsi aux inconvénients et dangers liés aux méthodes de l'art antérieur.

La présente invention a donc pour objet un procédé de préparation d'acides perfluoroalcanecarboxyliques et perfluoroalcanesulfoniques, caractérisé en ce que l'on fait réagir, dans un milieu protique, de l'ozone sur une chaîne perfluoroalkyle comportant au moins un groupe chimique oxydable.

Les chaînes perfluoroalkyles qui constituent donc les substrats de départ dans le cadre de la présente invention, peuvent comporter 1 à 12 atomes de carbone et présentent la formule générale

CF₃(CF₂)ₙY

dans laquelle Y représente le groupe chimique oxydable qui peut être choisi parmi :
- les composés de formule CF₂X, X étant un hétéroatome, de préférence un halogène, et plus préférentiellement encore l'iode, ou
- un groupe hétéroatomique, de préférence un groupe soufré, ou
- un groupe aromatique.

Selon un mode de réalisation préférentiel, le groupe soufré est choisi parmi les arylthioéthers, le groupe aryle étant choisi parmi les radicaux mono ou bicycliques, pouvant comporter au moins un substituant choisi parmi l'hydrogène, les radicaux alkyles linéaires ou ramifiés, les radicaux halogène, éther, alcoxy, aryloxy, carboxylate métallique, acyloxy, fluoroalkylthio, fluoroalcoxy et acide carboxylique.

Selon un autre mode de réalisation préférentiel de l'invention, le groupe aromatique est un radical aromatique mono ou bicyclique, pouvant comporter au moins un substituant choisi parmi l'hydrogène, les radicaux alkyles linéaires ou ramifiés, les radicaux halogène, éther, alcoxy, aryloxy, carboxylate métallique, acyloxy, fluoroalkyl, fluoroalcoxy et acide carboxylique.

Conformément au procédé selon l'invention, les réactions sont effectuées par simple mise en contact des substrats de départ avec des vapeurs contenant de l'ozone qui constitue l'élément chimique oxydant le plus puissant après le fluor, son potentiel normal d'oxydation par rapport à l'électrode normale à hydrogène étant de 2,07 V.

La réaction selon la présente invention est effectuée en milieu protique. Selon un mode de réalisation avantageux, le milieu protique est constitué par de l'eau, de l'alcool ou des acides carboxyliques, de préférence non fluorés, ou leurs mélanges.

La température de la réaction est comprise entre - 100° C et 100° C, préférablement entre 0 et 40° C. Plus préférentiellement encore, la réaction est effectuée à une température proche de la température ambiante.

En ce qui concerne les conditions de pression, la réaction est généralement effectuée à une pression inférieure à 10 Bars, plus préférentiellement à une pression proche de la pression atmosphérique.

De façon générale, le procédé selon l'invention peut donc être mis en oeuvre, avec une bonne efficacité dans des conditions douces, à température ambiante et à pression atmosphérique.

La réaction selon la présente invention peut aussi, selon un mode de réalisation avantageux, être effectuée en présence d'un produit solide insoluble présentant une grande surface spécifique tel que par exemple la silice, l'alumine, la silice-alumine, l'oxyde de titane, le charbon actif, les tourbes, les argiles, et les zéolithes. Ceci permet d'améliorer considérablement l'interaction entre l'ozone et les composés perfluorés.

Etant donné que les substrats de départ de formule CF₃(CF₂)ₙY sont très appauvris en électrons par les atomes de fluor, il est extrêmement surprenant et inattendu que cette oxydation puisse avoir lieu à une température proche de l'ambiante, sans aucune activation particulière.

En ce qui concerne les substrats de départ soufrés, l'art antérieur enseigne qu'ils ne sont oxydables en sulfones correspondants qu'à l'aide de réactifs très forts, tels que l'oxyde de chrome à chaud (R.M. Scribner, J. Org. Chem, 1966, 31 3671). Cette transformation n'affecte pas le noyau aromatique et ne produit pas les acides polyfluoroalcanesulfoniques. Par ailleurs, les sulfures usuels sont oxydés par l'ozone en sulfoxydes puis en sulfones. Ainsi, le méthylphénylsulfure est transformé en sulfone correspondante avec un rendement quantitatif (D. Barnard, J. Chem. Soc., 1957,4547). Il est donc particulièrement étonnant que l'oxydation par l'ozone, selon l'invention, des arylperfluoralkylsulfures se poursuive jusqu'à la formation des acides perfluoroalcanesulfoniques, alors que leur atome de soufre est moins oxydable que celui du méthylphénylsulfure.

Sans que cette théorie puisse être considérée comme limitative, il est possible que l'ozone attaque, en premier lieu, le noyau aromatique et non pas l'atome de soufre dans les substrats de départ comportant un groupe soufré.

Selon un mode de réalisation préféré de l'invention, le rapport molaire ozone/substrat est compris entre 1 et 20, de préférence entre 2 et 5 pour les acides perfluoroalcanecarboxyliques et entre 3 et 8 pour les acides perfluoroalcanesulfoniques.

Un cosolvant peut avantageusement être ajouté pour solubiliser le substrat de départ. Ce cosolvant peut être choisi parmi les solvants aprotiques tels que les nitriles, en particulier l'acétonitrile, les organochlorés, en particulier le tétrachlorométhane, les amines secondaires ou tertiaires, en particulier la diéthylamine, la triéthylamine et la tributylamine et les amines présentant une ou des chaînes alkyles plus longues.

La durée de la réaction selon l'invention peut varier entre environ trente minutes et sept jours.

Les exemples qui suivent ont pour but de mieux illustrer l'invention.

### Exemple 1

Dans un réacteur en verre, on introduit 20 g d'iodure de perfluorooctyle (C₈F₁₇I) et 150 ml d'acide propanoïque. Le mélange est agité à température ambiante et un balayage d'un mélange ozone/oxygène (6/94 volume sur volume) est appliqué dans l'atmosphère du récipient pendant 2 jours. Le précipité formé est alors filtré. L'acide perfluorooctanoïque (C₇F₁₅COOH) contenu dans le filtrat est précipité avec du pentane, filtré sur buchner et séché pour conduire à 5 g d'un solide blanc. Le rendement d'obtention de l'acide perfluorooctanoïque est donc de 30 %. Ce rendement a été calculé après analyse par RMN¹⁹F dans du CDCl₃ à 300 MHz, ce qui donne les résultats suivants :
δ(ppm) -80.9(3F, CF₃) ; -119.4(t, 2F,CF₂) ; -121.8(2F, CF₂); -122.2(2F,CF₂) ; -122.9(s, 2F, CF₂) ; -126.3(d, 2F, CF₂CF₃).

### Exemple 2

On procède comme dans l'exemple 1. Après douze heures de réaction à température ambiante, l'analyse du résidu indique la formation de l'acide perfluorohexanoïque (C₅F₁₁COOH), avec un rendement de 25 %. Ce rendement a été calculé après analyse par RMN ¹⁹F dans du CDCL₃ à 282 MHz, ce qui donne les résultats suivants :
δ(ppm) -80.8(t, 3F, CF₃) ; -116.5(2F, CF₂) ; -122.1(qd, 4F, 2CF₂) ; -125.7(t, 2F, CF₂CF₃).

### Exemple 3

Dans un tube de verre, 1 g soit 2,52 mmoles, de tridécafluorohexylbenzène (PhC₆F₁₃) est mélangé à 20 ml de méthanol. Le mélange est agité à température ambiante et un mélange ozone/oxygène (6/94 volume sur volume) est appliqué dans la solution pendant 12 heures. L'analyse du mélange indique la formation de l'acide perfluoroheptanoïque (C₆F₁₃COOH).

Une analyse par RMN ¹⁹F dans du CDCl₃ à 282 MHz a donné les résultats suivants :
δ(ppm) -80.54 (t, 3F, CF₃) ; -116.2(d, 2F, CF₂COOH) ; -121.16(d, 2F, CF₂) ; -121.9(d, 2F, CF₂) ; -122.15(d, 2F, CF₂); -125.6(2F, CF₂CF₃).

### Exemple 4

0.95 g de sulfure de perfluorobutyle et de phényle (2.89 mmoles) sont mélangés à 20 ml de méthanol et un équivalent de triéthylamine, soit 0,4 ml est ajouté. Le mélange ozone/oxygène (6/94 volume sur volume) est barbotté dans le liquide pendant 7 heures. Ensuite, un deuxième équivalent d'amine est ajouté et l'ozonolyse est poursuivie pendant 7 heures supplémentaires à température ambiante. On obtient un mélange 50/50 de l'acide carboxylique (C₃F₇COOH) et de l'acide sulfonique (C₄F₉SO₃H).

Une analyse par RMN ¹⁹F dans du CDCl₃ à 282MHz a donné les résultats suivants :
C₄F₉SO₃H
δ(ppm) -80.6(3F, CF₃) ; -114.1(2F, CF₂SO₃) ; -120.9(2F, CF₂); -125.4(2F, CF₂CF₃)
C₃F₇CO₂H
δ(ppm) -80.6(3F, CF₃) ; -117.0 (2F, CF₂COOH) ; -125.5(2F, CF₂CF₃).

### Exemple 5

1g, soit 5.6 mmoles, de phényltrifluorométhylsulfure est dissous dans 20 ml d'un mélange méthanol/eau (8/2 volume sur volume). Le mélange ozone/oxygène (6/94 volume sur volume) est barbotté dans le liquide réactionnel pendant six heures.

L'analyse du mélange indique la formation du trifluorométhanesulfonate de méthyle (CF₃SO₃Me) et de l'acide correspondant dans un rapport 3/1.

L'hydrolyse de l'ester s'effectue en présence de baryte pendant douze heures. L'excès de baryte est neutralisé et précipité en ajoutant de l'acide sulfurique dilué jusqu'à un pH légèrement acide. Puis l'acide trifluorométhanesulfonique résultant est précipité à l'aide de carbonate de Baryum.

Les solvants sont ensuite évaporés et le solide extrait en continu avec de l'acétone pendant six heures.

Une analyse par RMN ¹⁹F dans du CD₃OD à 282 MHz a donné les résultats suivants :
CF₃SO₃H δ(ppm) -78.2(3F, CF₃)
CF₃SO₂Me δ(ppm) -74.63(3F, CF₃)
(CF₃SO₃)₂Ba δ(ppm) -78.35(3F, CF₃)

### Exemple 6

1g, soit 5.6 mmoles, de trifluorométhylsulfure (PhSCF₃) est dissous dans 20 ml de méthanol. Une pointe de spatule (5 à 10 mg) de dioxyde de titane est ajoutée au mélange réactionnel. Le mélange ozone-oxygène (6/94 volume sur volume) est barbotté dans le liquide réactionnel pendant 4 heures.

Les pourcentages relatifs des produits formés sont évalués par R.M.N. du Fluor. Ainsi, l'ester méthylé de l'acide trifluorométhanesulfonique (CF₃SO₃H) est présent à raison d'environ 15 %, et l'acide trifluorométhanesulfonique est présent à raison d'environ 15 %.

### Exemple 7

1 g, soit 5.6 mmoles, de trifluorométhylsulfure (PhSCF₃) est dissous dans 20 ml d'un mélange méthanol-eau (9/1 volume sur volume ). Une pointe de spatule de silice est ajoutée au mélange réactionnel. Le mélange ozone-oxygène (6/94 volume sur volume) est barbotté dans le liquide réactionnel pendant 5 h 30.

Les pourcentages relatifs des produits formés sont évalués par R.M.N. du Fluor. Ainsi, l'ester méthylé de l'acide trifluorométhanesulfonique est présent à raison d'environ 60 % et l'acide trifluorométhanesulfonique est présent à raison d'environ 30 %.

## Revendications

1. Procédé de préparation d'acides perfluoroalcanecarboxyliques et perfluoroalcanesulfoniques, suivant lequel on fait réagir, dans un milieu protique, de l'ozone sur une chaîne perfluoroalkyle comportant au moins un groupe chimique oxydable, caractérisé par le fait que le groupe chimique oxydable est choisi parmi :
- un groupe de formule CF₂X, X étant un hétéroatome, de préférence un halogène, et plus préférentiellement encore l'iode,
- un groupe hétéroatomique, de préférence un groupe soufré à l'exclusion d'un groupe disulflure,
- un groupe aromatique.

2. Procédé selon la revendication 1, caractérisé en ce que le groupe soufré est choisi parmi les arylthioéthers, le groupe aryle étant choisi parmi les radicaux mono ou bicycliques, pouvant comporter au moins un substituant choisi parmi l'hydrogène, les radicaux alkyles linéaires ou ramifiés, les radicaux halogène, éther, alcoxy, aryloxy, carboxylate métallique, acyloxy, fluoroalkylthio, fluoroalcoxy, acide carboxylique.

3. Procédé selon la revendication 2, caractérisé en ce que le groupe aromatique est un radical aromatique mono ou bicylique, pouvant comporter au moins un substituant choisi parmi l'hydrogène, les radicaux alkyles linéaires ou ramifiés, les radicaux halogène, éther, alcoxy, aryloxy, carboxylate métallique, acyloxy, fluoroalkyl, fluoroalcoxy, acide carboxylique.

4. Procédé selon l'une quelconque des revendications 1 à 3, caractérisé en ce que le milieu protique est constitué par de l'eau, de l'alcool ou des acides carboxyliques, de préférence non fluorés, ou leurs mélanges.

5. Procédé selon l'une quelconque des revendications 1 à 4, caractérisé en ce que l'on ajoute dans le milieu un cosolvant aprotique choisi parmi les nitriles, en particulier l'acétonitrile, les organochlorés, en particulier le tétrachlorure de carbone, et les amines secondaires ou tertiaires, en particulier la diéthylamine, la triéthylamine et la tributylamine.

6. Procédé selon l'une quelconque des revendications 1 à 5, caractérisé en ce que la température est comprise entre -100° C et 100° C, de préférence entre 0 et 40° C, et plus préférentiellement encore proche de la température ambiante.

7. Procédé selon l'une quelconque des revendications 1 à 6, caractérisé en ce que la pression est inférieure à 10 Bars, et de préférence proche de la pression atmosphérique.

8. Procédé selon l'une quelconque des revendications 1 à 7, caractérisé en ce que le rapport molaire ozone/substrat est compris entre 1 et 20, de préférence entre 2 et 5 pour les acides perfluoroalcanecarboxyliques et entre 3 à 8 pour les acides perfluoroalcanesulfoniques.

9. Procédé selon l'une quelconque des revendications 1 à 8, caractérisé en ce que l'on ajoute un produit solide insoluble présentant une grande surface spécifique, choisi notamment parmi la silice, l'alumine, la silice-alumine, l'oxyde de titane, le charbon actif, les tourbes, les argiles, et les zéolithes.

## Claims

1. Process for the preparation of perfluoroalkanecarboxylic acids and perfluoroalkanesulfonic acids, according to which ozone is reacted, in a protic medium, with a perfluoroalkyl chain containing at least one oxidizable chemical group, characterized in that the oxidizable chemical group is chosen from:
- a group of formula CF₂X, X being a hetero atom, preferably a halogen, and even more preferably iodine,
- a heteroatomic group, preferably a sulfur-containing group, except for a disulfide group,
- an aromatic group.

2. Process according to Claim 1, characterized in that the sulfur-containing group is chosen from aryl thioethers, the aryl group being chosen from mono- or bicyclic radicals which can contain at least one substituent chosen from hydrogen, linear or branched alkyl radicals and halogen, ether, alkoxy, aryloxy, metal carboxylate, acyloxy, fluoroalkylthio, fluoroalkoxy and carboxylic acid radicals.

3. Process according to Claim 2, characterized in that the aromatic group is a mono- or bicyclic aromatic radical which can contain at least one substituent chosen from hydrogen, linear or branched alkyl radicals and halogen, ether, alkoxy, aryloxy, metal carboxylate, acyloxy, fluoroalkyl, fluoroalkoxy and carboxylic acid radicals.

4. Process according to any one of Claims 1 to 3, characterized in that the protic medium consists of water, of alcohol or of carboxylic acids, which preferably do not contain fluorine, or mixtures thereof.

5. Process according to any one of Claims 1 to 4, characterized in that an aprotic cosolvent chosen from nitriles, in particular acetonitrile, organochlorine compounds, in particular carbon tetrachloride, and secondary or tertiary amines, in particular diethylamine, triethylamine and tributylamine, is added to the medium.

6. Process according to any one of Claims 1 to 5, characterized in that the temperature is between -100°C and 100°C, preferably between 0 and 40°C and even more preferably close to room temperature.

7. Process according to any one of Claims 1 to 6, characterized in that the pressure is less than 10 bar and preferably close to atmospheric pressure.

8. Process according to any one of Claims 1 to 7, characterized in that the ozone/substrate molar ratio is between 1 and 20, preferably between 2 and 5, for the perfluoroalkanecarboxylic acids, and between 3 and 8 for the perfluoroalkanesulfonic acids.

9. Process according to any one of Claims 1 to 8, characterized in that an insoluble solid product with a large specific surface area, chosen in particular from silica, alumina, silica-alumina, titanium oxide, active charcoal, peats, clays and zeolites, is added.

## Patentansprüche

1. Verfahren zur Herstellung von Perfluoralkancarbon- und Perfluoralkansulfonsäuren, worin man in einem protischen Medium Ozon auf eine Perfluoralkylkette einwirken läßt, die wenigstens eine oxidierbare chemische Gruppe trägt, dadurch gekennzeichnet, daß die oxidierbare chemische Gruppe ausgewählt ist aus:
- einer Gruppe der Formel CF₂X, worin X ein Heteroatom, vorzugsweise Halogen und besonders bevorzugt Jod, bedeutet,
- einer heteroatomigen Gruppe, vorzugsweise einer schwefelhaltigen Gruppe mit Ausnahme einer Disulfidgruppe,
- einer aromatischen Gruppe.

2. Verfahren nach Anspruch 1, dadurch gekennzeichnet, daß die schwefelhaltige Gruppe ausgewählt ist aus Arylthioethern, wobei die Arylgruppe ausgewählt ist aus mono- oder bicyclischen Resten und wenigstens einen Substituenten tragen kann, der ausgewählt ist aus Wasserstoff, geradkettigen oder verzweigten Alkylresten, Halogen-, Ether-, Alkoxy-, Aryloxy-, Metallcarboxylat-, Acyloxy-, Fluoralkylthio-, Fluoralkoxy- und Carboxylresten.

3. Verfahren nach Anspruch 2, dadurch gekennzeichnet, daß die aromatische Gruppe ein mono- oder bicyclischer aromatischer Rest ist, der wenigstens einen Substituenten tragen kann, der ausgewählt ist aus Wasserstoff, geradkettigen oder verzweigten Alkylresten, Halogen-, Ether-, Alkoxy-, Aryloxy-, Metallcarboxylat-, Acyloxy-, Fluoralkoxy- und Carboxylresten.

4. Verfahren nach irgendeinem der Ansprüche 1 bis 3, dadurch gekennzeichnet, daß das protische Medium aus Wasser, Alkohol oder Carbonsäuren, vorzugsweise nicht-fluoriert, oder deren Mischungen gebildet wird.

5. Verfahren nach irgendeinem der Ansprüche 1 bis 4, dadurch gekennzeichnet, daß man in das Medium ein aprotisches Cosolvens gibt, das ausgewählt ist aus Nitrilen, insbesondere Acetonitril, Organochlorverbindungen, insbesondere Tetrachlorkohlenstoff, und sekundären oder tertiären Aminen, insbesondere Diethylamin, Triethylamin und Tributylamin.

6. Verfahren nach irgendeinem der Ansprüche 1 bis 5, dadurch gekennzeichnet, daß die Temperatur zwischen -100°C und 100°C, vorzugsweise zwischen 0 und 40°C und besonders bevorzugt nahe Umgebungstemperatur liegt.

7. Verfahren nach irgendeinem der Ansprüche 1 bis 6, dadurch gekennzeichnet, daß der Druck niedriger als 10 bar und vorzugsweise nahe Atmosphärendruck liegt.

8. Verfahren nach irgendeinem der Ansprüche 1 bis 7, dadurch gekennzeichnet, daß das Molverhältnis von Ozon/Substrat zwischen 1 und 20 liegt, vorzugsweise zwischen 2 und 5 für die Perfluoralkancarbonsäuren und zwischen 3 und 8 für die Perfluoralkansulfonsäuren.

9. Verfahren nach irgendeinem der Ansprüche 1 bis 8, dadurch gekennzeichnet, daß man einen unlöslichen Feststoff mit einer hohen spezifischen Oberfläche zugibt, der insbesondere ausgewählt ist aus Siliciumdioxid, Aluminiumoxid, Siliciumdioxid-Aluminiumoxid, Titandioxid, Aktivkohle, Torfen, Tonen und Zeolithen.
